(19) **Europäisches Patentamt / European Patent Office / Office européen des brevets**

(11) **EP 4 567 393 B1**

(12) **EUROPEAN PATENT SPECIFICATION**

(45) Date of publication and mention
of the grant of the patent:
**05.08.2026  Bulletin 2026/32**

(21) Application number: **23213911.3**

(22) Date of filing: **04.12.2023**

(51) International Patent Classification (IPC):
*G01L 9/00* (2006.01)      *G01L 19/00* (2006.01)

(52) Cooperative Patent Classification (CPC):
**G01L 9/0042; G01L 9/0054; G01L 19/0092; G01L 19/145**

(54) **PIEZORESISTIVE SENSOR ELEMENT AND PIEZORESISTIVE PRESSURE SENSOR WITH MINIMIZED LONG-TERM DRIFT**

PIEZORESISTIVES SENSORELEMENT UND PIEZORESISTIVER DRUCKSENSOR MIT MINIMIERTER LANGFRISTIGER DRIFT

ÉLÉMENT DE CAPTEUR PIÉZORÉSISTIF ET CAPTEUR DE PRESSION PIÉZORÉSISTIF AVEC DÉRIVE À LONG TERME RÉDUITE AU MINIMUM

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**11.06.2025  Bulletin 2025/24**

(73) Proprietors:
- **TE Connectivity Solutions GmbH**
  **8200 Schaffhausen (CH)**
- **First Sensor AG**
  **12459 Berlin (DE)**

(72) Inventors:
- **Jenni, Kaspar**
  **8200 Schaffhausen (CH)**

- **Teipen, Rafael**
  **12459 Berlin (DE)**
- **Jean-Mistral, Claire**
  **8200 Schaffhausen (CH)**
- **Nörthemann, Kai**
  **12459 Berlin (DE)**
- **Nikpourian, Alireza**
  **12459 Berlin (DE)**

(74) Representative: **Grünecker Patent- und Rechtsanwälte**
**PartG mbB**
**Leopoldstraße 4**
**80802 München (DE)**

(56) References cited:
EP-A1- 3 032 235      US-A1- 2022 048 761
US-B1- 7 856 885

**Description**

The present disclosure relates to piezoresistive sensors. In particular, the present disclosure relates to a piezoresistive sensor element and to a piezoresistive pressure sensor that detects mechanical stress acting on a membrane.

For instance, EP 3 032 235 A1 discusses a prior art pressure sensor having a first bridge with resistors located on a membrane for measuring the external pressure exerted on the membrane. This first bridge has a zero-offset due to temperature variations and due to package stress. This offset - may be compensated by using the output of a second bridge, having four resistors located outside the membrane. This total double bridge sensor provides a pressure value that is compensated for package stress, however, the compensation is not perfect.

US 7 856 885 B1 presents a MEMS-based silicon pressure sensor for the ocean environment. A multiple diaphragm piezoresistive pressure sensor for measuring the pressure of a liquid is described, comprising an inner deformable diaphragm formed on a silicon substrate, the inner deformable diaphragm having a first thickness an outer deformable diaphragm formed on the silicon substrate, the outer deformable diaphragm having a second thickness which is greater than the first thickness, positioned below the inner deformable diaphragm to support the inner deformable diaphragm, a first piezoresistive bridge embedded in the inner deformable diaphragm, a second piezoresistive bridge embedded in the outer deformable diaphragm and possibly a third piezoresistive bridge embedded in the silicon substrate to compensate for temperature variations.

US 2022/048761 A1 presents a MEMS pressure sensor comprising: a sensor portion comprising a deformable membrane and a first volume, and a valve portion comprising a first output to a first side of the pressure sensor and a second output to a second side of the pressure sensor. The valve portion is operable to close the second output and open the first output to equalize pressure in the first volume with pressure at the first side of the pressure sensor for calibrating the sensor; and close the first output and open the second output to equalize pressure in the first volume with pressure at the second side of the pressure sensor for pressure measurement.

The piezoresistive sensor element according to the present disclosure is based on the physical principle of piezoresistivity, which will be outlined shortly in the following.

Piezoresistive sensors are among the first Micro-Electro-Mechanical-Systems (MEMS) devices and comprise a substantial market share of MEMS sensors in the market today. In particular, silicon piezoresistance has been widely used for various sensors including pressure sensors, accelerometers, cantilever force sensors, inertial sensors, and strain gauges. A detailed overview is for instance given in Barlian, A. Alvin & Park, Woo-Tae & Mallon, Joseph R., Jr. & Rastegar, Ali J. & Pruitt, Beth L. (2009): "Review: Semiconductor Piezoresistance for Microsystems", Proceedings of the IEEE. Institute of Electrical and Electronics Engineers, 97, pp. 513-552, DOI: 10.1109/JPROC.2009.2013612.

It is known that the electrical resistance (R) of a homogeneous material is a function of its dimensions and resistivity ($\rho$),

$$R = \frac{\rho \cdot l}{a} \tag{1}$$

where l is length, and a is average cross-sectional area.

The change in resistance due to applied stress is a function of geometry and resistivity changes.

The cross-sectional area of a bulk material reduces in proportion to the longitudinal strain by its Poisson's ratio, v, which for most metals ranges from 0.20 to 0.35. For anisotropic silicon, the effective directional Poisson's ratio ranges from 0.06 to 0.36. The isotropic lower and upper limit for v are -1.0 and 0.5. The so-called gauge factor (GF) of a strain gauge is defined as

$$GF = \frac{\Delta R/R}{\varepsilon} \tag{2}$$

where $\varepsilon$ is strain and $\Delta R/R$ is fractional resistance change with strain. The change in resistance is due to both the geometric effects (1 + 2 v) and the fractional change in resistivity ($\Delta \rho/\rho$) of the material with strain

$$\frac{\Delta R}{R} = (1 + 2v)\varepsilon + \frac{\Delta \rho}{\varrho} \tag{3}$$

Geometric effects alone provide a GF of approximately 1.4 to 2.0, and the change in resistivity, $\Delta \rho/\rho$, for a metal is small-on the order of 0.3. However, for silicon and germanium in certain directions, $\Delta \rho/\rho$ is 50-100 times larger than the geometric term. For a semiconductor, elasticity and piezoresistivity are direction-dependent under specified directions of loads (stress, strain) and fields (potentials, currents). For the sensors according to the present disclosure, the stress induced change of resistivity (the so-called piezoresistive effect) is responsible for the generation of the electrical output signal.

In the following, some basics about the notation and fundamentals of piezoresistivity in semiconductors will be discussed.

As this is generally known, Miller indices can be used for describing crystal structure. Crystals have periodic arrangements of atoms arranged in one of 14 lattice types and complete reviews are available elsewhere. The Miller indices specify crystal planes by n-tuples. A direction index [hkl] denotes a vector normal to a plane described by (hkl), and t represents a family of planes equivalent to (hkl) by symmetry. Angle-bracketed indices, like (hkl), represent all directions equivalent to [hkl] by symmetry. In a hexagonal crystal, as found in most silicon carbide polytypes, the Bravais-Miller index scheme is commonly adopted where four indices are used to represent the intercept-reciprocals corresponding to the four principal crystal axes ($a_1$, $a_2$, $a_3$, and c). The axes $a_1$, $a_2$, and $a_3$ are on the same plane and 120° apart from one another while c is perpendicular to the a-plane defined by the ($a_1$, $a_2$, $a_3$) triplet.

Crystalline silicon forms a covalently bonded diamond-cubic structure with lattice constant a = 5.43 Å. The diamond-cubic structure is equivalent to two interpenetrating face-centered-cubic (FCC) lattices with basis atoms offset by 1/4a in the three orthogonal directions. Silicon's diamond-cubic lattice is relatively sparse (34% packing density) compared to a regular face-centered-cubic (FCC) lattice (74% packing density). Commonly used wafer surface orientations in micromachining include (100), (111), and (110).

Photolithography and etch techniques can create devices in various directions to access desirable material properties. For instance, a (111) oriented piezoresistor in a (110) plane will have the highest piezoresistive sensitivity in a pressure sensor. More commonly, (110) aligned piezoresistors on (100) wafers are used because of their high equal and opposite longitudinal and transverse piezoresistive coefficients.

To define the state of stress for a unit element, nine components, $\sigma_{ij}$, must be specified, as given in the following matrix:

$$\sigma = \begin{bmatrix} \sigma_{11} & \sigma_{12} & \sigma_{13} \\ \sigma_{21} & \sigma_{22} & \sigma_{23} \\ \sigma_{31} & \sigma_{32} & \sigma_{33} \end{bmatrix} \tag{4}$$

The first index i denotes the direction of the applied stress, while j indicates the direction of the force or stress. If i = j, the stress is normal to the specified surface, while i ≠ j indicates a shear stress on face i. From static equilibrium requirements that forces and moments sum to zero, a stress tensor is always symmetric, that is $\sigma_{ij} = \sigma_{ji}$, and thus the stress tensor contains only six independent components.

Strain, $\varepsilon_{ij}$, is also directional. For an isotropic, homogeneous material, stress is related to strain by Hooke's Law, $\sigma = \varepsilon E$. Although "effective" values of Young's modulus and Poisson's ratio for a single direction are often employed for simple loading situations, a tensor is required to fully describe the stiffness of an anisotropic material such as silicon. The stress and strain are related by the elastic stiffness matrix, C, where $\sigma_{ij} = C_{ijkl} * \varepsilon kl$, or equivalently by the inverse compliance matrix, S, where $\varepsilon_{ij} = S_{ijkl} * \sigma kl$:

$$\begin{bmatrix} \sigma_{11} \\ \sigma_{22} \\ \sigma_{33} \\ \sigma_{23} \\ \sigma_{13} \\ \sigma_{12} \end{bmatrix} = \begin{bmatrix} c_{11} & c_{12} & c_{13} & c_{14} & c_{15} & c_{16} \\ c_{21} & c_{22} & c_{23} & c_{24} & c_{25} & c_{26} \\ c_{13} & c_{23} & c_{33} & c_{34} & c_{35} & c_{36} \\ c_{14} & c_{24} & c_{34} & c_{44} & c_{45} & c_{46} \\ c_{15} & c_{25} & c_{35} & c_{45} & c_{55} & c_{56} \\ c_{16} & c_{26} & c_{36} & c_{46} & c_{56} & c_{66} \end{bmatrix} \begin{bmatrix} \varepsilon_{11} \\ \varepsilon_{22} \\ \varepsilon_{33} \\ 2\varepsilon_{23} \\ 2\varepsilon_{13} \\ 2\varepsilon_{12} \end{bmatrix} \tag{5}$$

$$\begin{bmatrix} \varepsilon_{11} \\ \varepsilon_{22} \\ \varepsilon_{33} \\ 2\varepsilon_{23} \\ 2\varepsilon_{13} \\ 2\varepsilon_{12} \end{bmatrix} = \begin{bmatrix} s_{11} & s_{12} & s_{13} & s_{14} & s_{15} & s_{16} \\ s_{21} & s_{22} & s_{23} & s_{24} & s_{25} & s_{26} \\ s_{13} & s_{23} & s_{33} & s_{34} & s_{35} & s_{36} \\ s_{14} & s_{24} & s_{34} & s_{44} & s_{45} & s_{46} \\ s_{15} & s_{25} & s_{35} & s_{45} & s_{55} & s_{56} \\ s_{16} & s_{26} & s_{36} & s_{46} & s_{56} & s_{66} \end{bmatrix} \begin{bmatrix} \sigma_{11} \\ \sigma_{22} \\ \sigma_{33} \\ \sigma_{23} \\ \sigma_{13} \\ \sigma_{12} \end{bmatrix} \tag{6}$$

Collapsed notation reduces each pair of subscripts to one number: 11→1, 22→2, 33→3, 23→4, 13→5, 12→6, e.g., $\sigma_{11}$ to $\sigma_1$, $\varepsilon_{12}$ to $\varepsilon_6$, $c_{1111}$ to $c_{11}$ and $s_{2323}$ to $s_{44}$.

Single crystal germanium and silicon, both of which have a diamond lattice crystal structure, were the first materials widely used as piezoresistors. Piezoresistive coefficients are used for describing the correlation between the electric field components, the current density and the stress. The piezoresistive coefficients ($\pi$) require four subscripts because they relate two second-rank tensors of stress and resistivity. The first subscript refers to the electric field component (measured potential), the second to the current density (current), and the third and fourth to the stress (stress has two directional components). For conciseness, the subscripts of each tensor are also collapsed, e.g., $\pi_{1111} \to \pi_{11}$, $\pi_{1122} \to \pi_{12}$, $\pi_{2323} \to \pi_{44}$. These relations may be generalized for a fixed voltage and current orientation ($\omega$) as a function of stress ($\lambda$):

$$\frac{\Delta \rho_\omega}{\rho} = \sum_{\lambda=1}^{6} \pi_{\omega\lambda}\, \sigma_\lambda$$

$$(7)$$

These coefficients were determined for relatively lightly doped silicon and germanium samples with resistivities ranging from 1.5-22.7 $\Omega$-cm, e.g., 7.8 $\Omega$-cm for p-type silicon. Current commercial and research practice uses doping levels several orders of magnitude higher. Higher concentrations have somewhat lower piezoresistive coefficients, but much lower temperature coefficients of resistance and sensitivity. For example, doping levels are known that result in resistivities in the range of 0.005-0.2 $\Omega$-cm. Piezoresistive coefficients have been measured for (100) samples along the (100) and (110) crystal directions. Longitudinal and transverse coefficients for the fundamental crystal axes were determined directly. Shear piezoresistive coefficients were inferred. By these measurements and considering the crystal symmetry, the piezoresistive tensor of 7.8 $\Omega$-cm silicon was fully characterized according to Smith, C.S. (1954) Piezoresistance Effect in Germanium and Silicon. Physical Review, 94, 42-49, as follows:

$$[\pi_{\omega\lambda}] = \begin{bmatrix} \pi_{11} & \pi_{12} & \pi_{12} & 0 & 0 & 0 \\ \pi_{12} & \pi_{22} & \pi_{12} & 0 & 0 & 0 \\ \pi_{12} & \pi_{12} & \pi_{33} & 0 & 0 & 0 \\ 0 & 0 & 0 & \pi_{44} & 0 & 0 \\ 0 & 0 & 0 & 0 & \pi_{44} & 0 \\ 0 & 0 & 0 & 0 & 0 & \pi_{44} \end{bmatrix} =$$

$$\begin{bmatrix} 6.6 & -1.1 & -1.1 & 0 & 0 & 0 \\ -1.1 & 6.6 & -1.1 & 0 & 0 & 0 \\ -1.1 & -1.1 & 6.6 & 0 & 0 & 0 \\ 0 & 0 & 0 & 138.1 & 0 & 0 \\ 0 & 0 & 0 & 0 & 138.1 & 0 \\ 0 & 0 & 0 & 0 & 0 & 138.1 \end{bmatrix} \times 10^{-11} Pa^{-1}$$

$$(8)$$

For a p-type piezoresistor implanted on (100) silicon along the (100) crystal direction, the transversal and longitudinal piezoresistive coefficients may be defined and approximated as follows:

$$\pi_t = \frac{1}{2}(\pi_{11} + \pi_{12} - \pi_{44}) = -\frac{\pi_{44}}{2} \qquad (9)$$

$$\pi_l = \frac{1}{2}(\pi_{11} + \pi_{12} + \pi_{44}) = \frac{\pi_{44}}{2} \qquad (10)$$

Thus, the resulting change in resistance is

$$\frac{\Delta R}{R} = \sigma_l \pi_l + \sigma_t \pi_t \qquad (11)$$

$$\frac{\Delta R}{R} = \pi_{44}(\sigma_l - \sigma_t) \qquad (12)$$

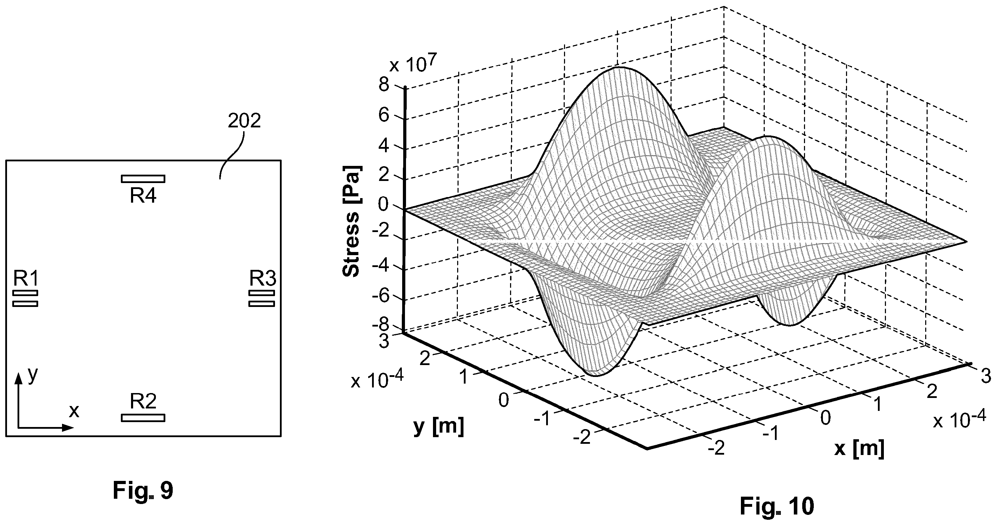

Fig. 10 illustrates a simulation of the stress values $\sigma_l$-$\sigma_t$ for a membrane 202 as depicted in Fig. 9 when a pressure of 1 bar is applied from the back. In this example, the membrane has dimensions of 390 $\mu$m in the x direction, 390 $\mu$m in the y direction, and a thickness of 6 $\mu$m.

It should be noted that a difference has to be made between the terms stress and strain.

When a material is put under pressure or has a mechanical load applied to it, mechanical stress is developed. When a solid is put under stress, it has the ability to deform. This deformation is called strain. The stress is the pressure per unit area of the material, and the resulting strain is the deformation that occurs as a result of this stress. Strain and stress are strongly intertwined because strain occurs solely as a result of stress. Stress is defined as the force per unit area generated within materials as a result of externally applied forces, unequal heating, or persistent deformation. The unit for stress is Nm$^{-2}$ (Pa). On the other hand, strain is defined as the amount of distortion experienced by the body in the direction of force compared to the original dimension of the object. Strain defines the relative change in the shape of an object.

The piezoresistors used for the piezoresistive sensor elements according to the present disclosure are responsive to the stress they experience.

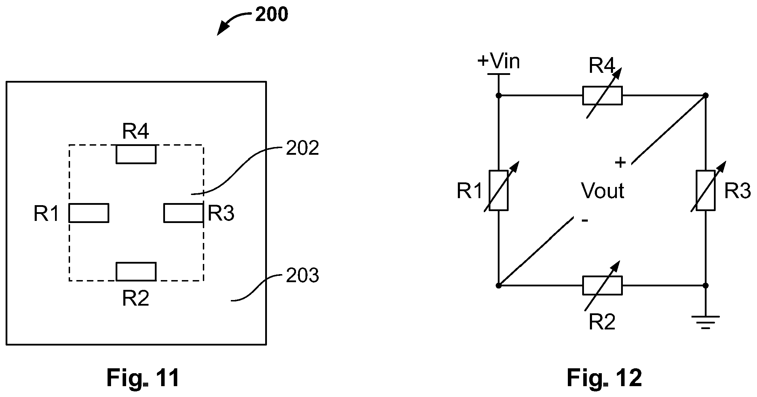

Furthermore, it is known in the art to use the concept of a Wheatstone bridge in order to ensure a precise and offset free measurement. Fig. 11 illustrates a schematic representation of a MEMS pressure sensor 200 comprising four stress sensitive piezoresistors R1, R2, R3, R4. A deflectable membrane (also referred to as diaphragm) 202 is surrounded by a stiffer frame 204. Pressure that acts on the membrane 202 causes stress in the membrane which is sensed by the four stress sensitive piezoresistors R1, R2, R3, R4.

These piezoresistors are arranged in the Wheatstone bridge circuit as depicted in Fig. 12 for a full-bridge configuration. When no pressure is applied, the output voltage, Vout is 0 V as all the piezoresistors are equal to $R_0(1+\alpha\Delta T)$. When the pressure is applied, the diaphragm is under stress. The active piezoresistors, which are positioned in the high stress regions on the diaphragm are strained, hence change their values to $R_0(1+\alpha\Delta T +\pi_l \Delta\sigma_l +\pi_t \Delta\sigma_t)$ where $\alpha$ is the temperature coefficient of resistance. $\pi_l$ and $\pi_t$ are the longitudinal and transverse piezoresistive coefficients, respectively. Meanwhile $\Delta T$, $\Delta\sigma_l$, and $\Delta\sigma_t$ are the changes in temperature, longitudinal stress and transverse stress, respectively. For example with pressure coming from the top of the membrane, the active perpendicular resistors (R1 and R3) experience mostly the longitudinal stress and the active parallel resistors (R2 and R4) undergo mostly the transverse stress. In other words, the resistors all experience both stresses. The sum of these two stresses is drawn in Figure 10, illustrating why two of these resistors increase in value while the two others decrease when the membrane is exposed to pressure. A longitudinal tensile stress increases the resistivity of p-type resistors, while a transverse stress has the opposite effect. Hence, the Vout changes accordingly due to these changes. Assume that $\Delta R1=\Delta R3$ and $\Delta R2=\Delta R4$, thus, the Vout expressions for the full-bridge (Fig. 13) are given by the following expression:

$$Vout = Vin\left(\frac{\pi_l\Delta\sigma_l-\pi_t\Delta\sigma_t}{2(1+\alpha\Delta T)+\pi_l\sigma_l-\pi_t\Delta\sigma_t}\right) \qquad (13)$$

As this is generally known, many disturbances that act uniformly on all four resistors can be eliminated by using the Wheatstone bridge configuration. Examples of such disturbances include process variations such as an implant dose, a linewidth and changes of the resistors due to temperature.

However, it has been found that conventional piezoresistive sensors suffer from slow long-term drift effects that are for instance caused by electrostatic build-up (for instance in a covering gel or a plastic cap) and/or environmental effects and/or process issues such as ionic contamination, material impurities, electromigration, stress release, stress in material layers, process residual stress, thermal coefficient (TC) mismatch, TC change etc. Stress isolation may cause such a drift, but this effect is not considered for the present disclosure because there exist concepts to limit this drift effect.

A slow drift of the offset caused by external disturbances cannot be discerned from slow changes of the measurand. In particular for the application of a MEMS pressure sensor, this drawback leads to a functional safety concern. In particular, relevant standards in the application fields of automotive industry, or safety in medical applications such as for respiratory devices In the context of the present disclosure, the term long-term drift relates to time spans between several months and about two years, preferably about one year.

Some conventional sensor concepts deal with this problem using a buried field shield for compensating the effect of any ionic contaminations due to the fabrication process. Further, it is known to use a polycrystalline silicon or metal field shield to nullify external electric fields. Some conventional sensors comprise a sensor housing having metal caps with lid shields in order to avoid electrostatic charge build-up. However, these counter-measures complicate the manufacturing process and often do not achieve satisfactory results.

Moreover, EP 3287758 B1 discloses a differential pressure sensor, which may provide a common mode corrected differential pressure reading. The differential pressure sensor includes for instance two pressure sensing diaphragms. The pressure sensor may be configured so that the first diaphragm measures the differential pressure between two sections of a fluid. The pressure sensor may also be configured so that the second diaphragm measures the common mode error experienced by the die at the time the differential pressure is read by the first diaphragm. Electrical connectors may be configured so that the differential pressure outputs a common mode error corrected differential pressure reading based on the readings of the first and second diaphragm. In particular, the second diaphragm includes at least one pressure sensitive electrical element that exhibits a varying resistance responsive to deflection of the diaphragm of the second pressure sensing die that is representative of a common mode error of the second pressure sensing die.

European patent application 22198016.2 relates to another differential pressure sensor. According to this document, the drift signal of the drift pressure sensor (i.e. a signal generated by a drift sensing unit formed on or in the symmetrical diaphragm) can additionally be used to trigger a warning signal. In more detail, by comparing the drift signal with a predefined threshold value, it is possible to estimate whether the differential pressure sensor (in particular a signal generated by a differential sensing unit formed on or the differential diaphragm) has been degraded.

This enables the technical effect that the differential pressure sensor can have a longer lifetime, in particular the pressure sensor does not need to be replaced after a predefined fixed time but can be replaced on demand (namely when the warning signal, which is sensed by the drift pressure sensor, is output).

However, there is still the need for a piezoresistive sensor, which allows a particularly precise and long-term stable measurement, at the same time being fabricated in a cost efficient manner.

This object is solved by the subject matter of the independent claim. Advantageous embodiments of the present disclosure are the subject matter of the dependent claims.

The present disclosure is based on the idea to provide a second Wheatstone bridge in addition to the main bridge for monitoring and compensating environmental and/or process induced drift effects. In particular, a piezoresistive sensor element according to the present disclosure comprises a substrate, which in operation is subjected to mechanical stress in response to a measurand to be measured, and a first array of at least four sensitive piezoresistors, wherein the sensitive piezoresistors are arranged on the substrate and are connected to form a first Wheatstone bridge for generating a first bridge signal. Further, a second array of at least four insensitive piezoresistors is provided, wherein the in-sensitive piezoresistors are connected to form a second Wheatstone bridge for generating a second bridge signal, and wherein the sensitive piezoresistors have a stress sensitivity which is higher than the stress sensitivity of the in-sensitive piezoresistors, and wherein an output signal of the piezoresistive sensor is generated based on a difference of the first and second bridge signals.

This solution has the advantage that by subtracting the output signals of the two bridges long-term drift can be compensated, while the sensitivity of the main bridge remains unaffected. Thus, the reliability and safety of the sensor is enhanced.

According to an advantageous example of the piezoresistive sensor element, the substrate comprises silicon, wherein each of the piezoresistors comprises doped areas ion-implanted in the silicon material. Silicon is a well-established material in the semiconductor industry and offers the potential for using established CMOS processes for fabricating the sensor element and further electronic components. As mentioned in the theoretical part above, monocrystalline silicon also exhibits a strong piezoresistive effect, in particular when using ion implantation for fabricating the piezoresistors.

However, it is clear that other materials showing a piezoresistive effect (for instance germanium or silicon carbide) may also be used in connection with the present disclosure. Further, for the fabrication of the piezoresistors, also other known technologies such as diffusion, epitaxy, or deposition of a doped polycrystalline silicon layer can also be employed.

When manufacturing the piezoresistive sensor element using a monocrystalline material, such as monocrystalline silicon, the piezoresistivity may be anisotropic. According to claim 1, each of the sensitive piezoresistors is oriented along a first crystallographic orientation of the substrate and each of the in-sensitive piezoresistors is oriented along a second crystallographic orientation of the substrate, the first crystallographic orientation being different from the second crystallographic orientation and causing a higher stress sensitivity than the second crystallographic orientation.

For instance, the substrate may be fabricated from p-type silicon with a (100) plane forming the outer surface, wherein the sensitive piezoresistors are arranged along a [$\overline{11}$0] direction or a [110] direction, and wherein the in-sensitive piezoresistors are arranged along a [100] direction or a [010] direction. A particularly high sensitivity and accuracy can be achieved with this configuration.

Alternatively, the substrate may also be fabricated from n-type silicon with a (100) plane forming the outer surface, wherein the sensitive piezoresistors are arranged along a [100] direction or a [010] direction or a [010] direction, and wherein the in-sensitive piezoresistors are arranged along a [110] direction or a [110] direction.

In order to ensure that the elements of the second Wheatstone bridge experience the same disturbances as the piezoresistors of the first Wheatstone bridge, so that all long-term drift effects match as closely as possible, each of the in-sensitive piezoresistors may be arranged in close proximity to one corresponding sensitive piezoresistor, so as to be subjected to essentially the same stress as the corresponding sensitive piezoresistor.

According to an advantageous example of the piezoresistive sensor element according to the present disclosure, the in-sensitive piezoresistors are arranged to include an angle of 45 degrees with each of the sensitive piezoresistors.

According to a further advantageous example, the piezoresistive sensor element further comprises a signal processing unit for evaluating the first bridge signal and the second bridge signal and for generating the sensor output signal. Thus, no external signal processing units and connection leads have to be involved for the compensation calculus, so that the accuracy and reliability can be increased.

The most accurate and compact architecture can be achieved when the signal processing unit is monolithically integrated with the piezoresistors on the same chip (also called die).

The effect of temperature influences may be monitored either be using the inherent temperature coefficient of resistance (TCR) of the second Wheatstone bridge or by additionally providing a temperature sensor, such as a temperature diode, arranged within the second Wheatstone bridge. Such a temperature diode is also known as a thermal diode. The functioning of a thermal diode is based on the property of electrical diodes to change voltage across it linearly according to temperature.

As mentioned above, the architecture proposed in the present disclosure is intended to eliminate the detrimental effects of long-term drift effects due to electrostatic build-up or external or process induced ionic influences. In order to enhance the effect of these influences on the second Wheatstone bridge, the passivation layer, which is present on the piezoresistive sensor element, may at least partly removed in regions above the in-sensitive piezoresistors.

The present disclosure relates exemplarily to a piezoresistive pressure sensor comprising at least one piezoresistive sensor element according to the principles described above, wherein the substrate comprises a deflectable membrane (which may also be referred to as a diaphragm), which in operation is deflected in response to a pressure to be measured.

It is clear that the ideas of the present disclosure may also be applicable to other piezoresistive sensors, such as force and inertial sensors. For instance, cantilever sensors, strain gauges, accelerometers, and gyroscopes may be equipped with piezoresistive sensor elements in line with the present disclosure. However, piezoresistive pressure sensors are some of the most reported and developed micromachined devices. Thus, the following detailed description will focus on piezoresistive pressure sensors, which typically measure deformation of a thin circular or rectangular membrane (diaphragm) under an applied external pressure. The membrane may be made from the same material as the wafer substrate (silicon, diamond, etc.) or CVD-based thin films (oxide, nitride, etc.). Integrated piezoresistors are formed by dopant diffusion, ion implantation, or doped epitaxy. However, it is clear that other materials showing a piezoresistive effect (for instance germanium or silicon carbide) may also be used in connection with the present disclosure.

According to an advantageous example, the membrane is surrounded by a frame having a higher stiffness than the membrane, and wherein the in-sensitive piezoresistors are arranged on the frame. Thus, the in-insensitive piezoresistors are decoupled from deformation of the membrane. The in-sensitive piezoresistors may in this case be arranged close to the periphery of the diaphragm or further away from the diaphragm. However, the in-sensitive piezoresistors are arranged on the deflectable membrane itself, so as to experience exactly the same stress and environment as the sensitive piezoresistors.

According to an advantageous example, the membrane has a rectangular outline and the sensitive piezoresistors have an elongated shape, and wherein a first pair of the sensitive piezoresistors are arranged along opposing sides of the deflectable membrane's outline and a second pair of the sensitive piezoresistors are arranged to include an angle with the other opposing sides of the deflectable membrane's outline.

The accompanying drawings are incorporated into the specification and form a part of the specification to illustrate several embodiments of the present disclosure. These drawings, together with the description serve to explain the principles of the disclosure. The drawings are merely for the purpose of illustrating the preferred and alternative examples of how the disclosure can be made and used, and are not to be construed as limiting the disclosure to only the illustrated and described embodiments. Furthermore, several aspects of the embodiments may formindividually or in different combinations-solutions according to the present disclosure. The following described embodiments thus can be considered either alone or in an arbitrary combination thereof. Further features and advantages will become apparent from the following more particular description of the various embodiments of the disclosure, as illustrated in the accompanying drawings, in which like references refer to like elements, and wherein:

**FIG. 1**    is a schematic top view of a piezoresistive pressure sensor according to a first example, outside the scope of the claims;

**FIG. 2**    is a schematic is a schematic cross sectional view of the piezoresistive pressure sensor shown in Fig. 1;

**FIG. 3**    is a schematic representation illustrating the crystallographic orientation of the piezoresistors according to an advantageous example, outside the scope of the claims;

**FIG. 4**    is a schematic representation of the electronic signal processing according to an advantageous example;

**FIG. 5**    is a schematic top view of a piezoresistive pressure sensor according to a second example, outside the scope of the claims;

**FIG. 6**    is a schematic is a schematic cross sectional view of a piezoresistive pressure sensor according to a further example;

**FIG. 7**    is a schematic top view of a piezoresistive pressure sensor according to a further example;

FIG. 8      is a schematic representation of the electronic signal processing according to a further advantageous example;

FIG. 9      is a schematic top view of a diaphragm region of a conventional piezoresistive pressure sensor;

FIG. 10     is schematic illustration of the stress distribution within the piezoresistive pressure sensor of Fig. 9;

FIG. 11     is a schematic top view of a conventional piezoresistive pressure sensor;

FIG. 12     is a circuit diagram of a Wheatstone bridge comprising the piezoresistors shown in Fig. 11.

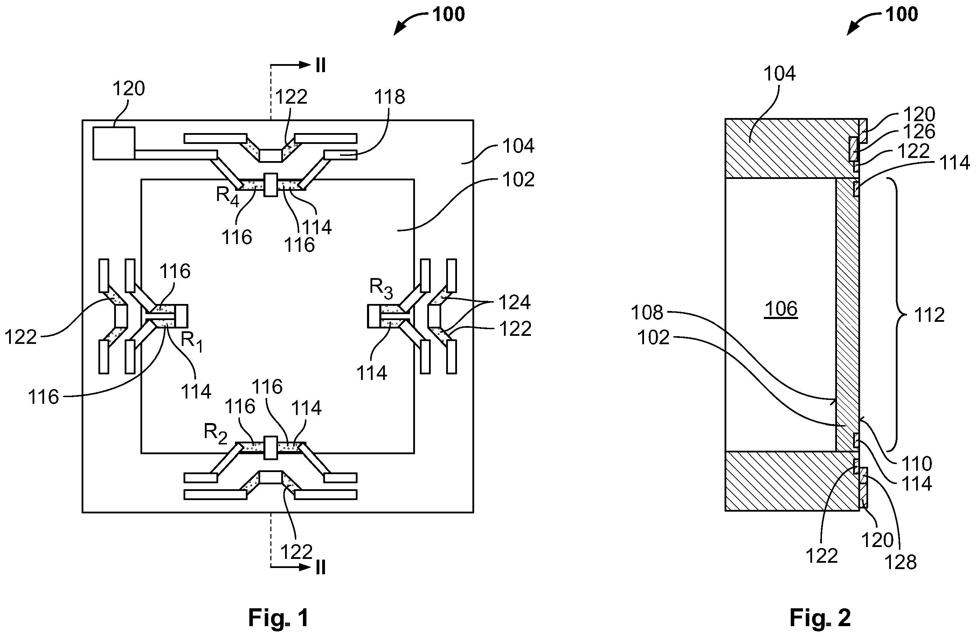

The present disclosure will now be explained in more detail with reference to the Figures and firstly referring to Fig. 1 and 2.

Fig. 1 is a schematic top view of a piezoresistive pressure sensor 100 according to a first example of the present disclosure, outside the scope of the claims. Figure 2 is the corresponding sectional view along the cut line II-II of Fig. 1.

According to this particular example, the piezoresistive pressure sensor 100 has a rectangular outline. Of course, the principles according to the present disclosure may also be applied to sensor structures with a different outline, e.g. a circular outline. The piezoresistive pressure sensor 100 comprises a deflectable membrane 102 which is also referred to as a diaphragm. The membrane 102 is surrounded and supported by a thicker and thus stiffer frame 104.

The membrane 102 and the frame 104 define a void 106, in which the pressure to be measured is allowed to act on the first (inner) side 108 of the membrane. The second side 110 of the membrane is oriented outwardly.

When a pressure difference is built up between the first side 108 and the second side 110 of the membrane 102, mechanical stress is generated. The membrane is deflectable and the maximal stress occurs on the edge. This may for instance be seen from Fig. 10. The maximum stress is therefore generated in the area 112 defined by the membrane 102. The frame 104 is the location where the stress caused by the pressure is minimal.

According to the present disclosure, a group of four sensitive piezoresistors 114 is arranged on the membrane 102 around the perimeter of the membrane 102. According to the shown example, each of the sensitive piezoresistors 114 comprises one or more, for instance two, ion implanted resistive regions 116 and electrically conductive connecting leads 118, which interconnect the resistive regions 116 and allow the interconnection between the sensitive piezoresistors 114 and to a signal processing circuit. Bond pads 120 may be provided for the electrical connection of the die for instance by wire bonding, solder bumps in flip-chip technology, or the like. In Fig. 1 and 2, only one of the pads 120 is shown in order to keep the schematic drawing simple.

The sensitive piezoresistors 114 in operation of the pressure sensor 100 change their resistivity in response to mechanical stress in the membrane with the maximum possible sensitivity. The four piezoresistors 114 are interconnected with each other to form a Wheatstone bridge circuit as explained above and shown in Fig. 12.

According to the present disclosure, in addition to these sensitive piezoresistors 114, a group of in-sensitive piezoresistors 122 is provided for compensating the effects of long-term drift. The insensitive piezoresistors 122 react towards mechanical stress with no or only a very low sensitivity. Thus, these in-sensitive piezoresistors 122 are essentially only perceptive of the drift causing influences. The in-sensitive piezoresistors 122 are connected to form a second Wheatstone bridge and the output signal of the second Wheatstone bridge can be subtracted from the output signal of the first Wheatstone bridge in order to yield an accurate and drift-compensated sensor signal.

The in-sensitive piezoresistors 122 may each comprise one or more interconnected ion implanted resistive regions 124.

As schematically illustrated in Fig. 2, the connecting leads 118 may for instance comprise diffused conductors 126 or deposited conductive layers, e. g. metal layers 128.

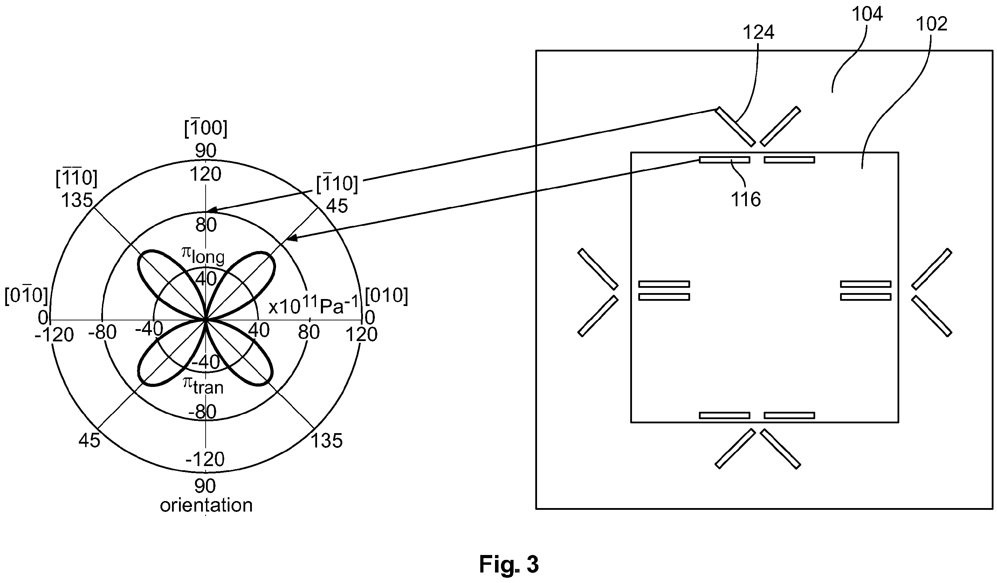

The example shown in Fig. 1 and 2 shows a piezoresistive pressure sensor 100, which is based on p-type monocrystalline silicon. As can be derived from Fig. 1, the resistive regions 124 of the stress in-sensitive piezoresistors 122 are laid out at an angle of 45 degrees compared to the resistive regions 116 of the stress sensitive piezoresistors 114. The reason for this arrangement will now be explained with reference to Fig. 3.

In particular, in Fig. 3 the orientation of the ion implanted resistive regions 116 of the stress sensitive piezoresistors 114 and the ion implanted resistive regions 124 of the stress in-sensitive piezoresistors 122 is compared to the piezoresistive coefficients in the (100) plane of a p-type silicon substrate as for instance shown in Barlian, A. Alvin & Park, Woo-Tae & Mallon, Joseph R., Jr. & Rastegar, Ali J. & Pruitt, Beth L. (2009): "Review: Semiconductor Piezoresistance for Microsystems", Proceedings of the IEEE. Institute of Electrical and Electronics Engineers, 97, pp. 513-552, DOI: 10.1109/JPROC.2009.2013612.

The ion implanted resistive regions 116 of the stress sensitive piezoresistors 114 are arranged along the [110] direction and the [110] direction, so that these piezoresistors exhibit the maximum possible stress sensitivity. In contrast thereto, the ion implanted resistive regions 124 of the stress in-sensitive piezoresistors 122 are arranged along the [100] direction and the [010] direction, thus exhibiting no or only a negligible stress sensitivity.

Although not shown in the Figures, it should be noted that for n-type silicon the sensitive piezoresistors have to be arranged along the [100] direction, the [010] direction or the [010] direction, whereas the in-sensitive piezoresistors are arranged along the [$\overline{1}$10] direction or the [110] direction, in order to achieve an analogous result.

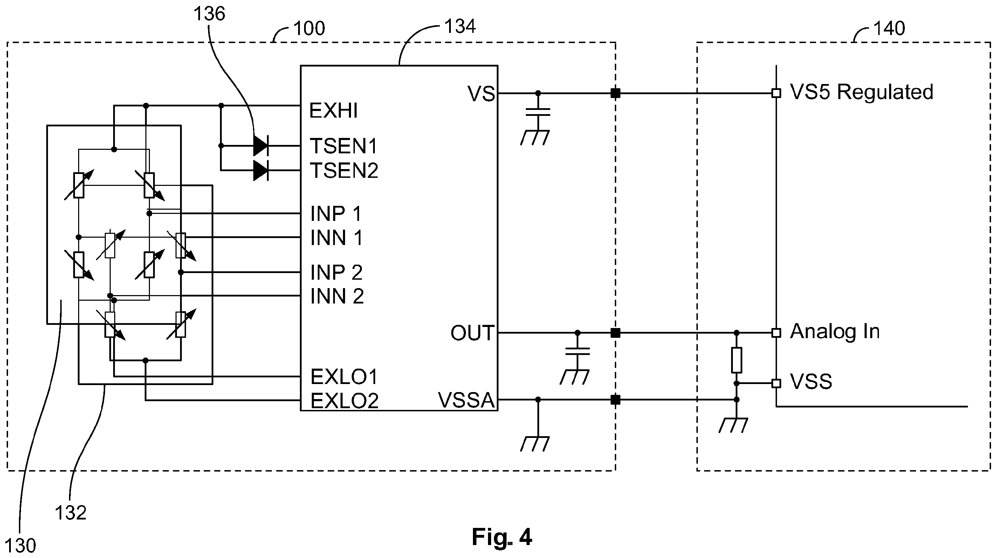

Fig. 4 illustrates an example of a sensor element 100, which integrally comprises the first Wheatstone bridge 130 with the sensitive piezoresistors, the second Wheatstone bridge 132 with the in-sensitive piezoresistors, and an integrated signal processing unit 134. The integrated signal processing unit 134 is operable to calculate a drift-compensated sensor output signal. Two temperature sensors (in particular, temperature diodes) 136 are provided for sensing the temperature of the sensor element 100, so that further compensation calculations can be performed, thus rendering the output signal even more accurate.

All components may for instance be integrated as one ASIC together with an electronic control circuit (ECU) 140.

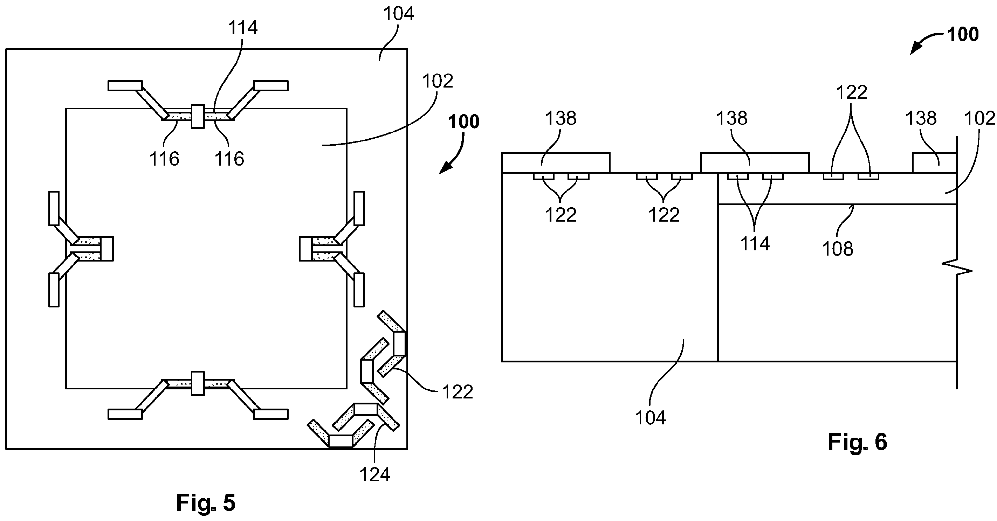

According to the example shown in Fig. 1 and 2, each of the in-sensitive piezoresistors 122 is placed in close vicinity to a corresponding sensitive piezoresistor 114. However, this does not necessarily have to be the case. The in-sensitive piezoresistors 122 may alternatively be located distanced away from the sensitive piezoresistors 114. An example of such an arrangement is depicted in Fig. 5, outside the scope of the claims. Here, the in-sensitive piezoresistors 122 are arranged in a peripheral region of the frame 104. Again, the resistive regions 124 of the stress in-sensitive piezoresistors 122 are laid out at an angle of 45 degrees compared to the resistive regions 116 of the stress sensitive piezoresistors 114. Thus, the stress sensitivity of in-sensitive piezoresistors 122 is zero or negligible compared to the stress sensitivity of the sensitive piezoresistors 114.

It has been found that the long-term drift effects that are to be tackled according to the present disclosure are partly screened by the usually applied passivation layers. The passivation for instance comprises a stack of a layer of silicon dioxide followed by a layer of silicon nitride. This finding is taken into account for the exemplary arrangement shown in Fig. 6.

According to Fig. 6, the passivation layer 138 is partly removed to augment the impact of the drift. In particular, the passivation layer 138 is removed from the regions where the in-sensitive piezoresistors 122 are located to facilitate the effects of the drift.

Furthermore, the backside 108 of the membrane 102 can also be a cause of drift due to electrical charges or due to leakage current, if the depth of the piezoresistors is close to the thickness of the membrane 102. As shown in Fig. 6, the in-sensitive piezoresistors 122 can be arranged on the frame 104 to make them as in-sensitive to stress as possible or on the membrane 102 to imitate the situation of the stress sensitive piezoresistors 114.

In Fig. 6, reference numeral 122 indicates stress insensitive resistors, whereas reference numeral 114 designates the stress sensitive piezoresistors. Another possibility would be to have the resistors 122 with a passivation on the membrane (i. e. the same arrangement as for the resistors 114, but in a stress insensitive orientation), but that arrangement would be less advantageous.

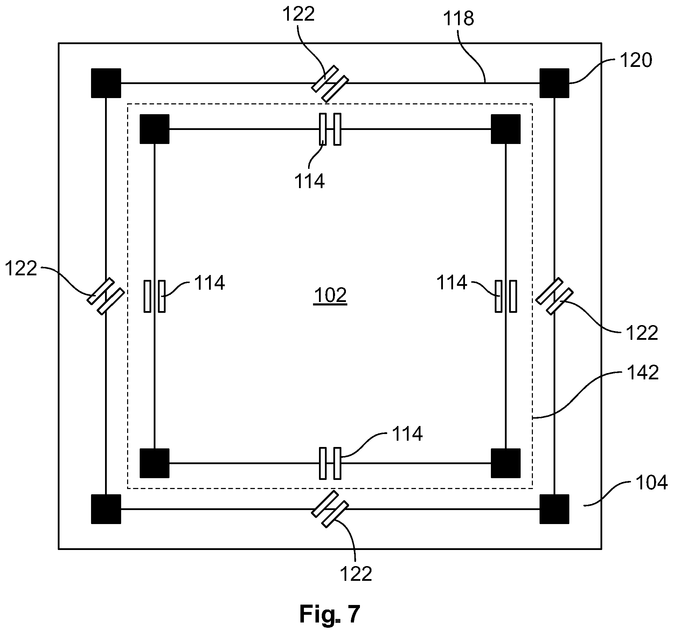

Fig. 7 illustrates still another advantageous example of a piezoresistive pressure sensor 100. The periphery of the membrane is marked here by a broken line 142. Again, the resistive regions of the stress in-sensitive piezoresistors 122 are laid out at an angle of 45 degrees compared to the resistive regions of the stress sensitive piezoresistors 114. Thus, the stress sensitivity of insensitive piezoresistors 122 is zero or negligible compared to the stress sensitivity of the sensitive piezoresistors 114.

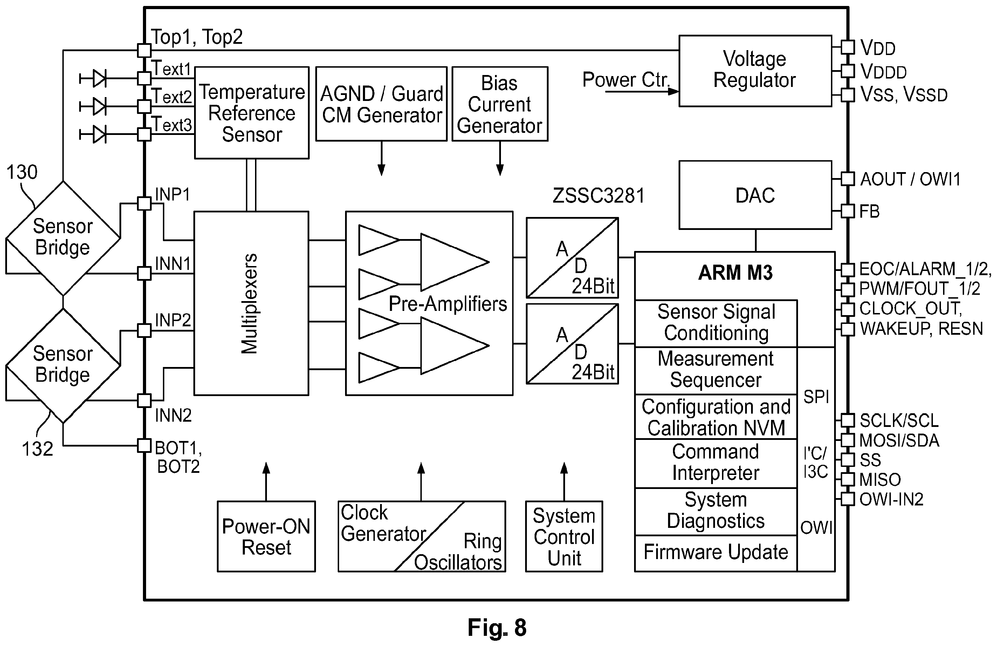

Fig. 8 illustrates a further example of an electronic circuit that can used for calculating the compensated sensor output signal from the output signals of the first Wheatstone bridge 130 with the sensitive piezoresistors and the second Wheatstone bridge 132 with the in-sensitive piezoresistors.

A compensation using an ASIC such as the dual channel 24-Bit resistive sensor signal conditioner with analog and digital output ZSSC3281sold by the company Renesas Electronics is a mathematical operation using the output of the stress sensitive bridge and stress insensitive bridge. An example is a subtraction of the stress insensitive output (i. e. the drift) from the stress sensitive output. Another example is to use the output of the stress insensitive bridge to trigger a warning when it exceeds a certain amount of drift.

**REFERENCE NUMERALS**

| Reference Numeral | Description |
| --- | --- |
| 100 | piezoresistive sensor element; pressure sensor |
| 102 | membrane, diaphragm |
| 104 | frame |
| 106 | void |
| 108 | first side of membrane, oriented towards void |
| 110 | second side of membrane, oriented towards outside of sensor |
| 112 | area with maximum stress |
| 114 | sensitive piezoresistor |

(continued)

| Reference Numeral | Description |
|---|---|
| 116 | ion implanted resistive region |
| 118 | connecting lead |
| 120 | contact pad |
| 122 | in-sensitive piezoresistor |
| 124 | ion implanted resistive region |
| 126 | diffused conductor |
| 128 | deposited metal layer |
| 130 | first Wheatstone bridge |
| 132 | second Wheatstone bridge |
| 134 | signal processing unit |
| 136 | temperature sensor |
| 138 | passivation |
| 140 | ECU |
| 142 | periphery of membrane |
| 200 | conventional piezoresistive pressure sensor |
| 202 | diaphragm |
| 204 | frame |

## Claims

1. Piezoresistive sensor element (100) comprising:

   a substrate, which in operation is subjected to mechanical stress in response to a measurand to be measured, a first array of at least four sensitive piezoresistors (114), wherein the sensitive piezoresistors (114) are arranged on the substrate and are connected to form a first Wheatstone bridge for generating a first bridge signal, and a second array of at least four in-sensitive piezoresistors (122), wherein the in-sensitive piezoresistors (122) are connected to form a second Wheatstone bridge for generating a second bridge signal,
   wherein the sensitive piezoresistors (114) have a stress sensitivity which is higher than the stress sensitivity of the in-sensitive piezoresistors (122),
   wherein an output signal of the piezoresistive sensor element (100) is generated based on a difference of the first and second bridge signals,
   **characterized in that**
   each of the sensitive piezoresistors (114) is oriented along a first crystallographic orientation of the substrate and each of the in-sensitive piezoresistors (122) is oriented along a second crystallographic orientation of the substrate,
   the first crystallographic orientation being different from the second crystallographic orientation and causing a higher stress sensitivity than the second crystallographic orientation.

2. Piezoresistive sensor element according to claim 1, wherein the substrate comprises silicon, and wherein each of the piezoresistors (114, 122) comprises doped areas ion-implanted in the silicon material.

3. Piezoresistive sensor element according to one of the preceding claims, wherein the substrate is fabricated from p-type silicon with a (100) plane forming the outer surface, and wherein the sensitive piezoresistors (114) are arranged along a [$\overline{1}$10] direction or a [110] direction, and wherein the in-sensitive piezoresistors (122) are arranged along a [100] direction or a [010] direction.

4. Piezoresistive sensor element according to one of the preceding claims, wherein the substrate is fabricated from n-type silicon with a (100) plane forming the outer surface, and wherein the sensitive piezoresistors (114) are arranged along a [100] direction or a [010] direction or a [010] direction, and wherein the in-sensitive piezoresistors (122) are arranged along a $[\overline{1}10]$ direction or a [110] direction.

5. Piezoresistive sensor element according to one of the preceding claims, wherein each of the in-sensitive piezoresistors (122) is arranged in close proximity to one corresponding sensitive piezoresistor (114), so as to be subjected to essentially the same stress as the corresponding sensitive piezoresistor (114).

6. Piezoresistive sensor element according to one of the preceding claims, wherein the insensitive piezoresistors (122) are arranged to include an angle of 45 degrees with each of the sensitive piezoresistors (114).

7. Piezoresistive sensor element according to one of the preceding claims, further comprising a signal processing unit (134) for evaluating the first bridge signal and the second bridge signal and for generating the sensor output signal.

8. Piezoresistive sensor element according to claim 7, wherein the signal processing unit (134) is monolithically integrated with the piezoresistors (114, 122).

9. Piezoresistive sensor element according to one of the preceding claims, further comprising a temperature sensor (136) arranged within the second Wheatstone bridge.

10. Piezoresistive sensor element according to one of the preceding claims, further comprising at least one passivation layer (138), wherein the passivation layer (138) is at least partly removed in regions above the in-sensitive piezoresistors (122).

11. Piezoresistive pressure sensor comprising:

a piezoresistive sensor element (100) according to one of the preceding claims, wherein the substrate comprises a deflectable membrane (102), which in operation is deflected in response to a pressure to be measured.

12. Piezoresistive pressure sensor according to claim 11, wherein the membrane (102) is surrounded by a frame (104) having a higher stiffness than the membrane (102), and wherein the in-sensitive piezoresistors (122) are arranged on the frame (104).

13. Piezoresistive pressure sensor according to claim 11, wherein the membrane (102) is surrounded by a frame (104) having a higher stiffness than the membrane (102), and wherein the in-sensitive piezoresistors (122) are arranged on the deflectable membrane (102).

14. Piezoresistive pressure sensor according to one of the claims 11 to 13, wherein the membrane (102) has a rectangular outline and the sensitive piezoresistors (114) have an elongated shape, and wherein a first pair of the sensitive piezoresistors (114) are arranged along opposing sides of the deflectable membrane's outline and a second pair of the sensitive piezoresistors (114) are arranged to include an angle with the other opposing sides of the deflectable membrane's outline.

**Patentansprüche**

1. Piezoresistives Sensorelement (100), das umfasst:

ein Substrat, das in Funktion in Reaktion auf eine zu messende Messgröße mechanischer Spannung ausgesetzt wird,
eine erste Anordnung aus wenigstens vier empfindlichen Piezoresistoren (114), wobei die empfindlichen Piezoresistoren (114) auf dem Substrat angeordnet und so verbunden sind, dass sie eine erste Wheatstone-Brücke zum Erzeugen eines ersten Brücken-Signals bilden, sowie
eine zweite Anordnung aus wenigstens vier unempfindlichen Piezoresistoren (122), wobei die unempfindlichen Piezoresistoren (122) so verbunden sind, dass sie eine zweite Wheatstone-Brücke zum Erzeugen eines zweiten Brücken-Signals bilden,
wobei die empfindlichen Piezoresistoren (114) eine Spannungsempfindlichkeit aufweisen, die höher ist als die Spannungsempfindlichkeit der unempfindlichen Piezoresistoren (122),
und ein Ausgangssignal des piezoresistiven Sensorelementes (100) auf Basis einer Differenz des ersten und des zweiten Brücken-Signals erzeugt wird,
**dadurch gekennzeichnet, dass**
jeder der empfindlichen Piezoresistoren (114) entlang einer ersten Kristallorientierung des Substrats angeordnet ist, und jeder der unempfindlichen Piezoresistoren (122) entlang einer zweiten Kristallorientierung des Substrats angeordnet ist,
wobei die erste Kristallorientierung sich von der zweiten Kristallorientierung unterscheidet und eine höhere Spannungsempfindlichkeit als die der zweiten Kristallorientierung bewirkt.

2. Piezoresistives Sensorelement nach Anspruch 1, wobei das Substrat Silizium umfasst und wobei jeder der Piezoresistoren (114, 122) dotierte Bereiche umfasst, die in das Siliziummaterial ionenimplantiert sind.

3. Piezoresistives Sensorelement nach einem der vorangehenden Ansprüche, wobei das Substrat aus p-leitendem Silizium hergestellt wird, bei dem eine (100)-Ebene die Außenfläche bildet,

und wobei die empfindlichen Piezoresistoren (114) entlang einer [110]-Richtung oder einer [110]-Richtung angeordnet sind,
und wobei die unempfindlichen Piezoresistoren (122) entlang einer [100]-Richtung oder einer [010]-Richtung angeordnet sind.

4. Piezoresistives Sensorelement nach einem der vorangehenden Ansprüche, wobei das Substrat aus n-leitendem Silizium hergestellt ist, bei dem eine (100)-Ebene die Außenfläche bildet,
und wobei die empfindlichen Piezoresistoren (114) entlang einer [100]-Richtung oder einer [010]-Richtung oder einer [010]-Richtung angeordnet sind, und wobei die unempfindlichen Piezoresistoren (122) entlang einer [1̄1̄0]-Richtung oder einer [110]-Richtung angeordnet sind.

5. Piezoresistives Sensorelement nach einem der vorangehenden Ansprüche, wobei jeder der unempfindlichen Piezoresistoren (122) in enger Nähe zu einem entsprechenden empfindlichen Piezoresistor (114) so angeordnet ist, dass er im Wesentlichen der gleichen Spannung wie der entsprechende empfindliche Piezoresistor (114) ausgesetzt wird.

6. Piezoresistives Sensorelement nach einem der vorangehenden Ansprüche, wobei die unempfindlichen Piezoresistoren (122) so angeordnet sind, dass sie einen Winkel von 45° mit jedem der empfindlichen Piezoresistoren (114) einschließen.

7. Piezoresistives Sensorelement nach einem der vorangehenden Ansprüche, das des Weiteren eine Signalverarbeitungs-Einheit (134) umfasst, mit der das erste Brücken-Signal sowie das zweite Brücken-Signal bewertet werden und das Sensor-Ausgangssignal erzeugt wird.

8. Piezoresistives Sensorelement nach Anspruch 7, wobei die Signalverarbeitungs-Einheit (134) monolithisch in die Piezoresistoren (114, 122) integriert ist.

9. Piezoresistives Sensorelement nach einem der vorangehenden Ansprüche, das des Weiteren einen Temperatursensor (136) umfasst, der innerhalb der zweiten Wheatstone-Brücke angeordnet ist

10. Piezoresistives Sensorelement nach einem der vorangehenden Ansprüche, das des Weiteren wenigstens eine Passivierungsschicht (138) umfasst, wobei die Passivierungsschicht (138) in Bereichen oberhalb der unempfindlichen Piezoresistoren (122) wenigstens teilweise entfernt ist.

11. Piezoresistiver Drucksensor, der umfasst:
ein piezoresistives Sensorelement (100) nach einem der vorangehenden Ansprüche, wobei das Substrat eine durchbiegbare Membran (102) umfasst, die in Funktion in Reaktion auf einen zu messenden Druck durchgebogen wird.

12. Piezoresistiver Drucksensor nach Anspruch 11, wobei die Membran (102) von einem Rahmen (104) umgeben ist, der eine höhere Steifigkeit hat als die Membran (102), und wobei die unempfindlichen Piezoresistoren (122) an dem Rahmen (104) angeordnet sind.

**13.** Piezoresistiver Drucksensor nach Anspruch 11, wobei die Membran (102) von einem Rahmen (104) umgeben ist, der eine höhere Steifigkeit hat als die Membran (102), und wobei die unempfindlichen Piezoresistoren (122) auf der durchbiegbaren Membran (102) angeordnet sind.

**14.** Piezoresistiver Drucksensor nach einem der Ansprüche 11 bis 13, wobei die Membran (102) einen rechteckigen Umriss hat und die empfindlichen Piezoresistoren (114) eine längliche Form haben, und wobei ein erstes Paar der empfindlichen Piezoresistoren (114) an einander gegenüberliegenden Seiten des Umrisses der durchbiegbaren Membran angeordnet sind und ein zweites Paar der empfindlichen Piezoresistoren (114) so angeordnet sind, dass sie einen Winkel mit den anderen einander gegenüberliegenden Seiten des Umrisses der durchbiegbaren Membran einschließen.

**Revendications**

**1.** Élément de capteur piézorésistif (100) comprenant :

un substrat qui, en fonctionnement, est soumis à une contrainte mécanique en réponse à un mesurande à mesurer,
un premier réseau d'au moins quatre piézorésistances sensibles (114), dans lequel les piézorésistances sensibles (114) sont disposées sur le substrat et sont connectées pour former un premier pont de Wheatstone destiné à générer un premier signal de pont, et
un second réseau d'au moins quatre piézorésistances insensibles (122), dans lequel les piézorésistances insensibles (122) sont connectées pour former un second pont de Wheatstone destiné à générer un second signal de pont,
dans lequel les piézorésistances sensibles (114) présentent une sensibilité à la contrainte qui est supérieure à la sensibilité à la contrainte des piézorésistances insensibles (122),
dans lequel un signal de sortie de l'élément de capteur piézorésistif (100) est généré sur la base d'une différence entre les premier et second signaux de pont,
**caractérisé en ce que**
chacune des piézorésistances sensibles (114) est orientée le long d'une première orientation cristallographique du substrat et chacune des piézorésistances insensibles (122) est orientée le long d'une seconde orientation cristallographique du substrat,
la première orientation cristallographique étant différente de la seconde orientation cristallographique et conférant une sensibilité à la contrainte supérieure à celle de la seconde orientation cristallographique.

**2.** Élément de capteur piézorésistif selon la revendication 1, dans lequel le substrat comprend du silicium, et dans lequel chacune des piézorésistances (114, 122) comprend des zones dopées implantées par implantation ionique dans le matériau de silicium.

**3.** Élément de capteur piézorésistif selon l'une des revendications précédentes, dans lequel le substrat est fabriqué à partir de silicium de type p avec un plan (100) formant la surface extérieure, et dans lequel les piézorésistances sensibles (114) sont disposées le long d'une direction [110] ou d'une direction [110], et dans lequel les piézorésistances insensibles (122) sont disposées le long d'une direction [100] ou d'une direction [010].

**4.** Élément de capteur piézorésistif selon l'une des revendications précédentes, dans lequel le substrat est fabriqué à partir de silicium de type n avec un plan (100) formant la surface extérieure, et dans lequel les piézorésistances sensibles (114) sont disposées le long d'une direction [100] ou d'une direction [010] ou d'une direction [010], et dans lequel les piézorésistances insensibles (122) sont disposées le long d'une direction [110] ou d'une direction [110].

**5.** Élément de capteur piézorésistif selon l'une des revendications précédentes, dans lequel chacune des piézorésistances insensibles (122) est disposée à proximité immédiate d'une piézorésistance sensible (114) correspondante, de manière à être soumise essentiellement à la même contrainte que la piézorésistance sensible (114) correspondante.

**6.** Élément de capteur piézorésistif selon l'une des revendications précédentes, dans lequel les piézorésistances insensibles (122) sont disposées de manière à former un angle de 45 degrés avec chacune des piézorésistances sensibles (114).

7. Élément de capteur piézorésistif selon l'une des revendications précédentes, comprenant en outre une unité de traitement de signal (134) destinée à évaluer le premier signal de pont et le second signal de pont et à générer le signal de sortie de capteur.

8. Élément de capteur piézorésistif selon la revendication 7, dans lequel l'unité de traitement de signal (134) est intégrée monolithiquement avec les piézorésistances (114, 122).

9. Élément de capteur piézorésistif selon l'une des revendications précédentes, comprenant en outre un capteur de température (136) disposé au sein du second pont de Wheatstone.

10. Élément de capteur piézorésistif selon l'une des revendications précédentes, comprenant en outre au moins une couche de passivation (138), dans lequel la couche de passivation (138) est au moins partiellement retirée dans des régions situées au-dessus des piézorésistances insensibles (122).

11. Capteur de pression piézorésistif comprenant :
un élément de capteur piézorésistif (100) selon l'une des revendications précédentes, dans lequel le substrat comprend une membrane fléchissable (102) qui, en fonctionnement, est fléchie en réponse à une pression à mesurer.

12. Capteur de pression piézorésistif selon la revendication 11, dans lequel la membrane (102) est entourée d'un cadre (104) présentant une rigidité supérieure à celle de la membrane (102), et dans lequel les piézorésistances insensibles (122) sont disposées sur le cadre (104).

13. Capteur de pression piézorésistif selon la revendication 11, dans lequel la membrane (102) est entourée d'un cadre (104) présentant une rigidité supérieure à celle de la membrane (102), et dans lequel les piézorésistances insensibles (122) sont disposées sur la membrane fléchissable (102).

14. Capteur de pression piézorésistif selon l'une des revendications 11 à 13, dans lequel la membrane (102) présente un contour rectangulaire et les piézorésistances sensibles (114) présentent une forme allongée, et dans lequel une première paire des piézorésistances sensibles (114) est disposée le long de côtés opposés du contour de la membrane fléchissable et une seconde paire des piézorésistances sensibles (114) est disposée de manière à former un angle avec les autres côtés opposés du contour de la membrane fléchissable.

100

104

120
126
122
114

112

110
114

128
120

122

104

106

108
102

122

**Fig. 2**

100

104
102

118

120

II

122

114
116

R₄

116

124
122

R₃

114

116

R₁

114

116

114
116

116

R₂

122

II

122

116

**Fig. 1**

**Fig. 3**

VS5 Regulated

Analog In

VSS

140

VS

OUT

VSSA

134

EXHI

TSEN1

TSEN2

INP 1

INN 1

INP 2

INN 2

EXLO1

EXLO2

100

136

132

130

**Fig. 4**

**Fig. 6**

**Fig. 5**

**Fig. 7**

EP 4 567 393 B1

**Fig. 8**

Fig. 10

Fig. 9

R3

R4

+Vin

R1

Vout

+

−

R2

**Fig. 12**

200

202

203

R4

R3

R1

R2

**Fig. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3032235 A1
- US 7856885 B1
- US 2022048761 A1
- EP 3287758 B1
- EP 22198016

**Non-patent literature cited in the description**

- **BARLIAN, A. ALVIN** ; **PARK, WOO-TAE** ; **MALLON, JOSEPH R., JR.** ; **RASTEGAR, ALI J.** ; **PRUITT, BETH L.** Review: Semiconductor Piezoresistance for Microsystems. *Proceedings of the IEEE. Institute of Electrical and Electronics Engineers*, 2009, vol. 97, 513-552
- **SMITH, C.S.** Piezoresistance Effect in Germanium and Silicon. *Physical Review*, 1954, vol. 94, 42-49